# EUROPEAN PATENT APPLICATION

(11) **EP 2 742 965 A1**
(43) Date of publication of application: **18.06.2014**
(21) Application number: 12821801.3
(22) Date of filing: 07.08.2012
(51) Int. Cl.: A61M 25/00

(54) **TREATMENT TOOL**

(30) Priority: 08.08.2011 US 201161572286 P
(71) Applicant: OLYMPUS MEDICAL SYSTEMS CORP., Tokyo 151-0072 (JP)
(72) Inventor: YAMATANI, Ken, Tokyo 151-0072 (JP); KAJI, Kunihide, Tokyo 151-0072 (JP)
(74) Representative: Schicker, Silvia
(86) International application number: PCT/JP2012/070116
(87) International publication number: WO 2013/022005

(57) **Abstract**

A endoscopic device has a longitudinal member having a longitudinal axis, a bending portion formed on a distal end side of the longitudinal member, a lumen formed along the longitudinal axis of the longitudinal member, and a through hole that communicate with the lumen, and opens toward the inner side of a bend of the bending portion when the bending portion is bent.

## Description

### Field of the Invention

The present invention relates to an endoscopic device that collects a liquid in a body.

Priority is claimed on United States Patent Application No. 61/572,286 provisionally applied in the United States on August 08, 2011, the content of which is incorporated herein by reference.

### Description of Related Art

In the related art, a catheter is known as an endoscopic device that suctions tissues or liquids in a body. For example, a catheter that has a plurality of suction passages formed in an outer surface thereof is disclosed in Patent Document 1. The catheter disclosed in Patent Document 1 has four suction passages that are punched in an outer surface of the catheter and that lead to an internal lumen, and blood or other fluids are suctioned into the inside of the catheter through the plurality of suction passages.

### [Prior Art Document]

### Patent Literature

[Patent Document 1] Published Japanese Translation No. 2009-537254 of the PCT International Publication

However, in the above Patent Document 1, because the plurality of suction passages are spirally arranged, for example, the suction passages that are oriented in the same direction collects the liquid and There is a possibility that the remaining the suction passages suction the tissues. Therefore, in Patent Document 1, it is difficult to fully collect the liquid from the suction passages. An object of the present invention is to provide an endoscopic device that can collect tissue more efficiently.

### SUMMARY OF THE INVENTION

The present invention suggests the following aspects in order to solve the above problems.

According to a first aspect of the present invention, an endoscopic device includes a longitudinal member which has a longitudinal axis; a bending portion which is formed on a distal end side of the longitudinal member; a lumen which is formed along the longitudinal axis of the longitudinal member; a through hole which communicates with the lumen and opens toward an inner side of a bend of the bending portion.

According to a second aspect of the present invention, in the first aspect, a connection port that is connected to a suction portion that suctions a liquid through an inside of the longitudinal member may be attached to a proximal end side of the longitudinal member.

According to a third aspect of the present invention, in the second aspect, the bending portion may have a predetermined restoring force that bends the bending portion so that the through hole is directed to the inner side of the bend of the bending portion.

According to a fourth aspect of the present invention, in the second aspect or the third aspect, the longitudinal member may have openings at the distal end and the proximal ends, respectively, the proximal end of the longitudinal member may be provided with an operating portion formed with a wire passage for inserting a stylet into the longitudinal member, and the connection port may branch from the passage and open to an external surface of the operating portion.

According to a fifth aspect of the present invention, in the third aspect, the bending portion may be spirally formed.

According to a sixth aspect of the present invention, in the third aspect, the endoscopic device may further include a support member that is connected to a delivery instrument that deliveries to the inside of the body and supports the delivery instrument at closer to a predetermined position of the proximal end than the curved portion.

According to a seventh aspect of the present invention, in the third aspect, the bending portion may be provided on the distal end side of the longitudinal member, and the longitudinal member may be provided with a curved portion formed such that a central axis of the longitudinal member is curved closer to the proximal end side than the bending portion.

According to an eighth aspect of the present invention, in the third aspect, the longitudinal member may have flexibility and be insertable through a channel of an endoscope.

According to a ninth aspect of the present invention, in the fifth aspect, the bending portion may be provided on the distal end side of the longitudinal member and may be formed in a conical coil shape.

According to a tenth aspect of the present invention, in the ninth aspect, a positional relationship between a central axis of the longitudinal member closer to the proximal end side than the bending portion and a centerline of the bending portion may be an intersecting or twisted position.

According to a eleventh aspect of the present invention, in the tenth aspect, a balloon may be provided on at least one of the proximal end side of the bending portion and the distal end side of the bending portion, the longitudinal member may be inserted into a digestive tract, and the balloon may inflate larger than the internal diameter of the lumen tissue by supplying a fluid thereinto.

### Effects of the Invention

According to the endoscopic device of the above respective aspects, the surface directed to the outer side of the bend in the external surfaces of the bending portion is pressed against the tissue, so that the depression can be formed in the tissue and a liquid can be collected through the through holes on the inner side of the bend. As a result, since a liquid can be collected in the depression, the liquid can be efficiently collected.

Moreover, since the through holes opens to the inner side of the bend of the bending portion, the openings of the through holes are not closed by the tissue, and a liquid can be efficiently collected. Additionally, according to the endoscopic device of the present embodiment, the tissue can also be prevented from being suctioned through the through holes.

### Brief Description of the Drawings

FIG. 1 is a side view showing an endoscopic device of a first embodiment of the present invention.
FIG. 2 is a view showing the operation of the endoscopic device of the first embodiment of the present invention.
FIG. 3 is a view showing the operation of the endoscopic device of the first embodiment of the present invention.
FIG. 4 is a view showing the operation of the endoscopic device of the first embodiment of the present invention.
FIG. 5 is a view showing the operation of the endoscopic device of the first embodiment of the present invention.
FIG. 6 is a view showing the operation of the endoscopic device of the first embodiment of the present invention.
FIG. 7 is a view showing the operation of the endoscopic device of the first embodiment of the present invention.
FIG. 8 is a side view showing the configuration of a modified example of the first embodiment of the present invention.
FIG. 9 is a view showing a usage example of the endoscopic device of the modified example of the first embodiment of the present invention.
FIG. 10 is a view showing a usage example of the endoscopic device of the modified example of the first embodiment of the present invention.
FIG. 11 is a view showing a usage example of the endoscopic device of the modified example of the first embodiment of the present invention.
FIG. 12 is a view showing a usage example of the endoscopic device of the modified example of the first embodiment of the present invention.
FIG. 13 is a view showing a usage example of the endoscopic device of the modified example of the first embodiment of the present invention.
FIG. 14 is a side view showing an endoscopic device of a second embodiment of the present invention.
FIG. 15 is a side view showing the configuration of a modified example of the second embodiment of the present invention.
FIG. 16 is a side view showing an endoscopic device of a third embodiment of the present invention.
FIG. 17 is a view as seen from arrow A of FIG. 16.
FIG. 18 is a side view showing the configuration and operation in use, of the endoscopic device of the fourth embodiment of the present invention.
FIG. 19 is a side view showing another configuration example of the endoscopic device of the first embodiment.

### Description of Embodiments

### (First Embodiment)

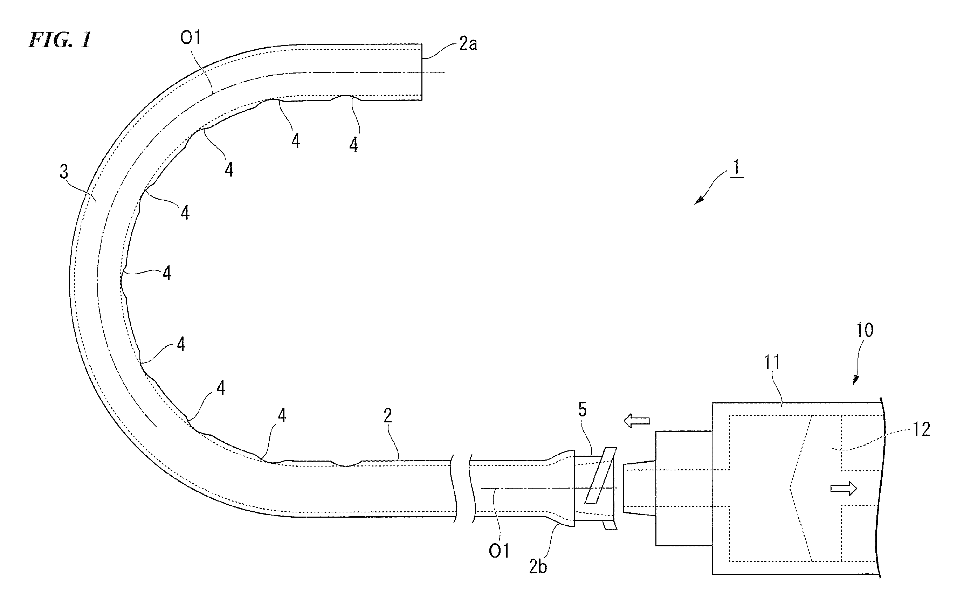

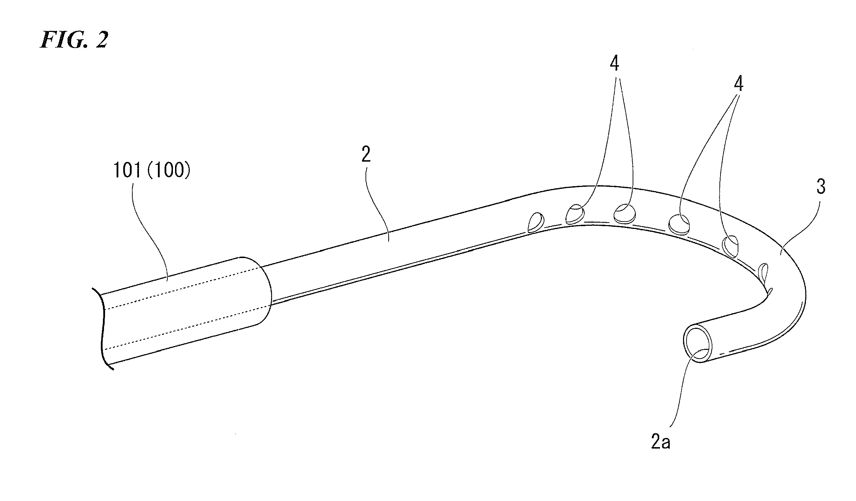

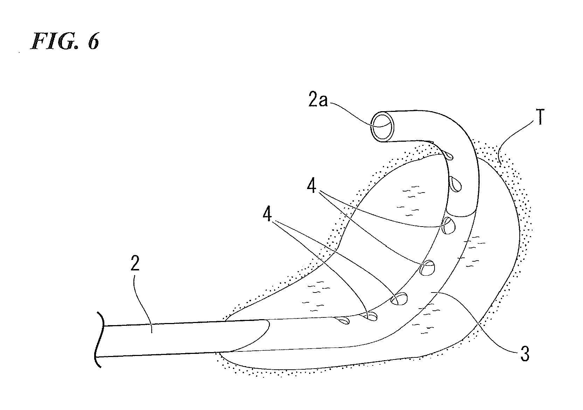

An endoscopic device 1 of a first embodiment of the present invention will be described below with reference to FIGS. 1 to 3. FIG. 1 is a side view showing the endoscopic device of the present embodiment. FIGS. 2 to 7 are views showing the operation of the endoscopic device.

First, the configuration of the endoscopic device 1 of the present embodiment will be described.

As shown in FIG. 1, the endoscopic device 1 includes a tube member 2 and a connection port 5.

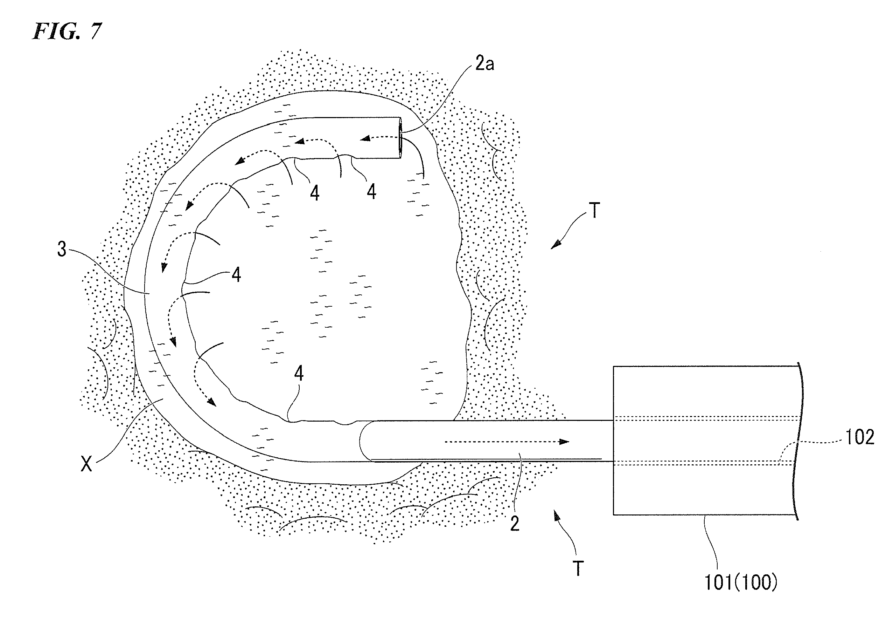

The tube member 2 has a longitudinal member. The tube member 2 is a tubular member that has flexibility and has an external diameter such that the tube member is insertable through a channel 102 (refer to FIG. 7) of an endoscope 100 as a delivery instrument to the inside of the body partially shown in FIG. 2. A distal end 2a of the tube member 2 and a proximal end 2b of the tube member 2 each open, and the internal space (lumen) of the tube member 2 is a flow channel through which a liquid in the body flows. Moreover, the tube member 2 has the bending portion 3 formed in a bent shape on the distal end 2a side. Additionally, a plurality of through holes 4 are provided in an outer wall of the bending portion 3, and communicates with the internal space.

The tube member 2 is transparent at least in a part or preferably the whole of the vicinity of the distal end 2a.

The bending portion 3 is formed by a bending tendency being given to the tube member 2. The shape of the bending portion 3 is a shape such that the bending portion is bent in a U-shape in an unloaded state and such that an opening formed at the distal end 2a of the tube member 2 is directed to the proximal end 2b side. When the bending portion 3 is inserted through the channel 102 shown in FIG. 7, the whole tube member 2 including the bending portion 3 deforms elastically in a shape along a bent state of an inner surface of the channel 102.

The plurality of through holes 4 are arranged side by side along the central axis O1 of the tube member 2, and when the bending portion 3 is bent, the through holes open toward the inner side of a bend of the bending portion 3.

The connection port 5 is a tubular member that communicates with the opening of the proximal end of the tube member 2, and has a lure lock structure or the like. A distal end of a syringe 11 of the syringe 10 (suction portion) corresponding to a lure lock is attached to the connection port 5 when the endoscopic device 1 is used. The endoscopic device 1 may have a configuration in which the tube member does not have the connection port 5, and the syringe 11 is directly attached to the proximal end opening of the tube member 2 or may have a configuration in which the proximal end opening of the tube member 2 is directly connected to a connection port of a suction pump.

Next, the operation of the endoscopic device 1 will be described.

The endoscopic device 1 of the present embodiment is inserted into the body, and is used in order to collect liquids accumulated in the body.

As shown in FIG. 1, when the endoscopic device 1 is used, the syringe 11 to which the plunger 12 is attached is attached to the connection port 5 of the endoscopic device 1.

During treatment, first, an operator performs delivery to a treatment target, for example, using the endoscope 100 (refer to FIG. 2) or the like, and observes the inside of the body of a patient who is the treatment target. If a liquid to be collected in the body is captured within a visual field of the endoscope 100, the operator inserts the tube member 2 of the endoscopic device 1 into the channel 102 from the distal end 2a.

The tube member 2 deforms elastically in a shape along the inner surface of the channel 102 within the channel 102 due to the flexibility of the tube member 2. If the tube member 2 is pushed into the channel 102, the distal end 2a of the tube member 2 is pushed out of the distal end of the channel 102 (refer to FIG. 2). Accordingly, as shown in FIG. 2, after the bending portion 3 provided at the distal end 2a of the tube member 2 is pushed out of the distal end of the channel 102, the bending portion is restored to its original bent shape.

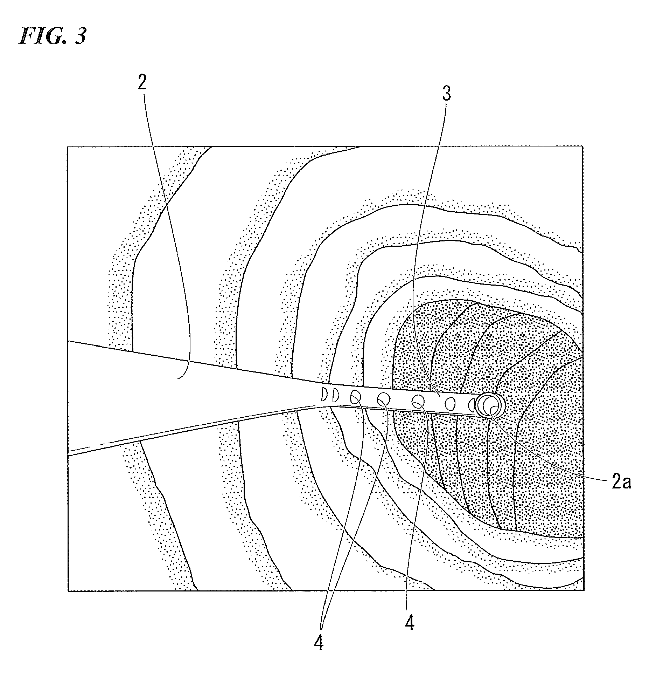

FIG. 3 is a schematic view of an image acquired using the endoscope 100. As shown in FIG. 3, the image of the endoscopic device 1 is acquired by the endoscope 100. Since the bending portion 3 is bent at this time, a surface located on the inner side of the bend is imaged by the endoscope 100. That is, the respective through holes 4 that open toward the inner side of the bend of the bending portion 3 is easily viewed by the operator who sees the endoscopic device 1 using the endoscope 100.

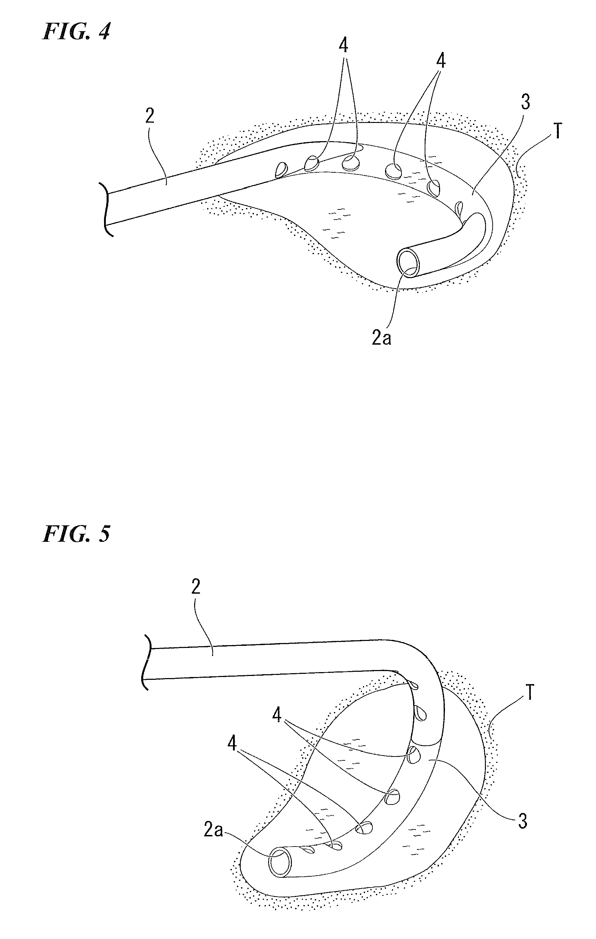

The operator brings the bending portion 3 into contact with the surface of a tissue T by moving the endoscope 100 in the body, moving the tube member 2 with respect to the endoscope 100, or operating the angle of the endoscope 100. For example, as shown in FIGS. 4 to 6, the operator moves the bending portion 3 to a region in where a liquid is accumulated, and appropriately adjusts the orientation of the bending portion 3 such that a portion of the bending portion 3 enters the liquid. Any position that is the distal end side, the intermediate portion, or the proximal end side of the bending portion 3 may be brought into contact with the tissue T. Accordingly, the plurality of through holes 4 formed in the bending portion 3 are arranged in the liquid.

Since the bending portion 3 has a bent shape, the distal end 2a of the tube member 2 is directed to a direction away from the surface of the tissue T in any orientations shown in FIGS. 4 to 6. For this reason, the distal end 2a of the tube member 2 is not pressed against the tissue T. An outside surface of the bend of the bending portion 3 contacts the tissue T. Since this outside surface is a surface that is smoothly bent, the outside surface does not damage the tissue T even if the outside surface is moved in a state where it is pressed against the tissue T.

If the bending portion 3 is pressed against the surface of the tissue T, the pliable tissue T is be pushed and deformed by the bending portion 3, and as shown in FIG. 7, a depression X is formed. Since the pliability of the tissue T varies depending on regions, the size of the depression X varies depending on regions.

A liquid in the body may be dammed by pleats in the surface of the tissue T. If the above depression X is formed by the bending portion 3, the pleats of the tissue T is smoothed out, whereby a liquid in the body is gathered in the depression X.

Additionally, as a method of forming the depression X, there is also a method of using the restoring force of the bending portion 3. Specifically, there is a method of forming the depression X when the bending portion 3 is restored to its original bent shape while pressing the tissue T. According to this method, even in a narrow lumen tissue where the angle of the endoscope 100 cannot be operated, the depression X can be formed in the surface of the tissue T and a liquid can be accumulated.

Moreover, if the pressing the tissue T is also used together by the restoring force of the aforementioned bending portion 3 when the bending portion 3 is pressed against the surface of the tissue T by the angle operation of the endoscope 100, the depression X can be formed with a larger force. This enables the depression X to be also formed on the surface of the fiberized hard tissue T, for example.

The operator can move the bending portion 3 along the surface of the tissue T in a state where the bending portion 3 is pressed against the surface of the tissue T if necessary. Accordingly, the pleats of the tissue T can be smoothed and a liquid can be moved to a desired position. At this time, since the outside surface of the bend of the bending portion 3 is a smooth surface without the opening formed at the distal end 2a of the tube member 2 or the openings of the through holes 4, there is no concern that the tissue T is damaged.

The operator tows the plunger 12 with respect to the syringe 11 as shown in FIG. 1 while maintaining a state where the depression X is formed by the bending portion 3. Accordingly, a liquid gathered in the depression X moves to the inside of the tube member 2 through the through holes 4, and are further collected within the syringe 11 through the inside of the tube member 2. Since the vicinity of the distal end 2a of the tube member 2 is transparent, the operator can view a liquid to be suctioned into the tube member 2 via the endoscope 100.

If all or a required amount of liquid is collected into the syringe 11, the tube member 2 is removed from the channel 102. Thereafter, if the observation or the like using the endoscope 100 is completed, the endoscope 100 is removed from the inside of the body. The endoscope 100 and the tube member 2 may be integrally removed from the inside of the body.

As described above, according to the endoscopic device 1 of the present embodiment, the surface directed to the outer side of the bend in the external surfaces of the bending portion 3 is pressed against the tissue T, so that the depression X can be formed in the tissue T and a liquid can be collected through the through holes 4 on the inner side of the bend. As a result, since a liquid can be collected in the depression X, the liquid can be efficiently collected.

Additionally, since the bending portion 3 of the tube member 2 are formed with the through holes 4, all the through holes 4 can be arranged in the depression X. For this reason, when a liquid is gathered within the depression X, the openings of substantially all the through holes 4 are located below the level of the liquid. As a result, the amount of the external air that is suctioned through the through holes 4 can be suppressed to be low, and the suction efficiency of a liquid can be enhanced.

Moreover, since the through holes 4 opens to the inner side of the bend of the bending portion 3, the openings of the through holes 4 are not closed by the tissue T, and a liquid can be efficiently collected. Additionally, according to the endoscopic device 1 of the present embodiment, the tissue T can also be prevented from being suctioned through the through holes 4.

In addition, the endoscopic device 1 of the present embodiment can favorably collect liquids accumulated in lumen tissues, such as an alimentary canal, a bile duct, a blood vessel, and a ureter, or saccate regions, such as a cyst. Additionally, according to the endoscopic device 1 of the present embodiment, even in regions other than the aforementioned tissues, liquids adhering to the tissue T that has pliability can be collected.

In addition, in the present embodiment, the endoscope 100 has been described as an example as the delivery instrument. However, if the delivery instrument is an instrument that can perform delivery to the inside of the body, such as an overtube, the delivery instrument is not particularly limited.

### (Modified Example)

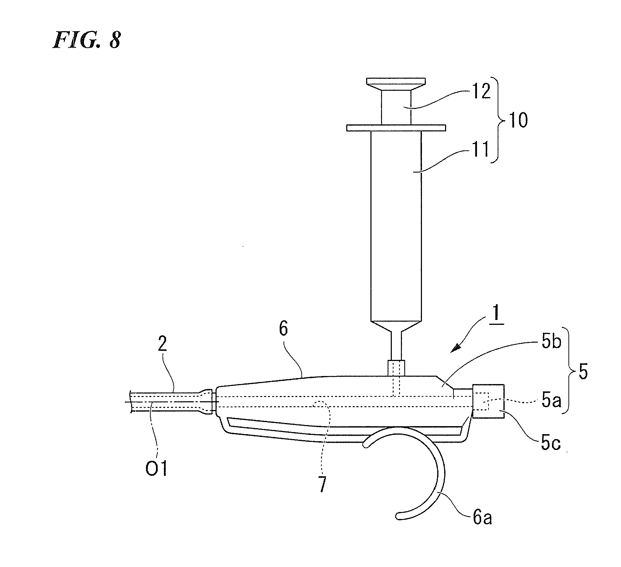

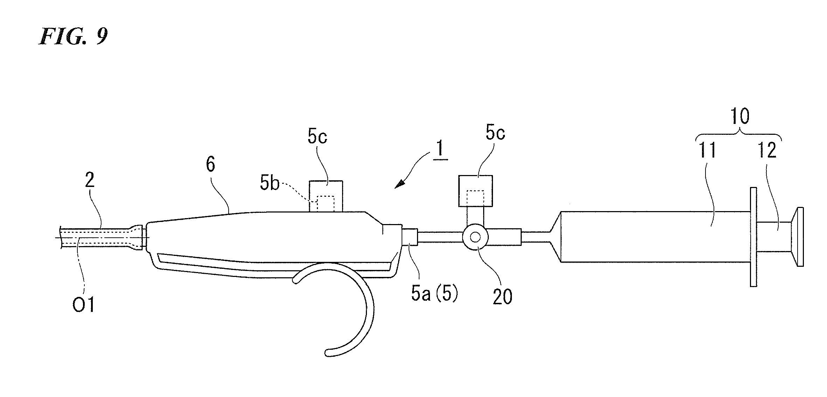

A modified example of the endoscopic device 1 of the above-described first embodiment will be described. FIG. 8 is a side view showing the configuration of the endoscopic device of the present modified example. FIGS. 9 to 13 are views showing usage examples of the endoscopic device of the present modified example.

As shown in FIG. 8, the present modified example is different from the endoscopic device of the above first embodiment in that the proximal end of the tube member 2 includes an operating portion 6 formed with the connection port 5 and a passage 7. The operating portion 6 may have a hook 6a or the like that can be attached to, for example, an operating portion (not shown) of the endoscope 100.

The connection port 5 includes a first connection port 5a that protrudes toward a proximal end side of the operating portion 6, and a second connection port 5b that protrudes toward a side of the operating portion 6. In the present modified example, the first connection port 5a has a cylindrical shape that is coaxial with the passage 7, and the second connection port 5b has a cylindrical shape that branches from the passage 7 and opens to an external surface of the operating portion 6. The first connection port 5a and the second connection port 5b have a configuration in which the syringe 11 can be attached to any of the connection ports. Additionally, the first connection port 5a and the second connection port 5b have a configuration in which the connection ports can be closed by a cap 5c when not used.

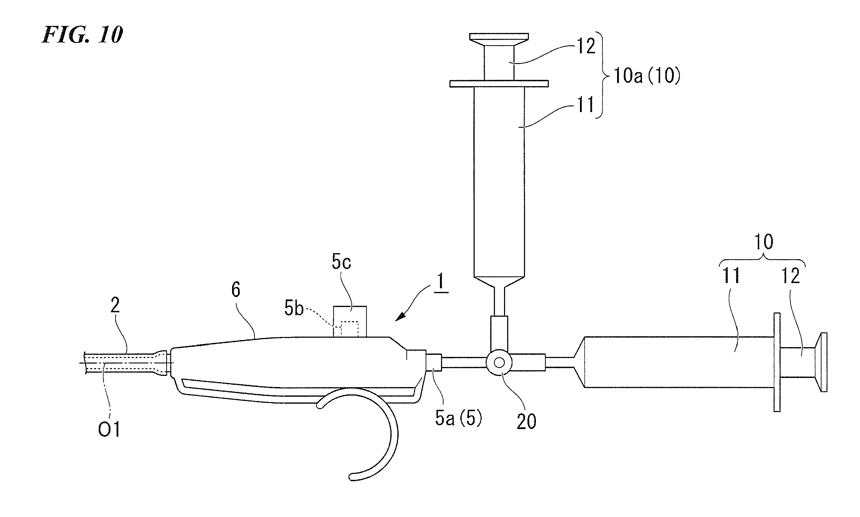

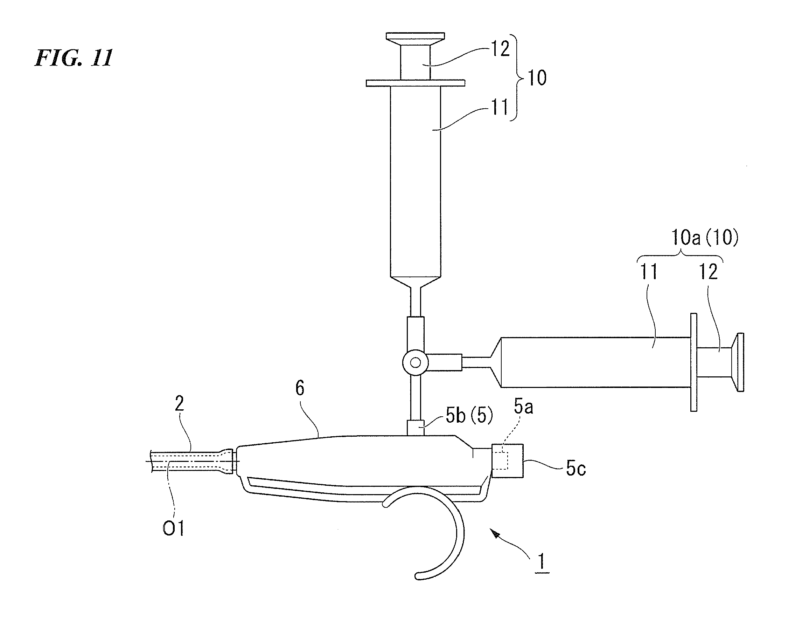

As shown in FIG. 9, the syringe 10 may be connected to the connection port 5 via a branch adapter 20. For example, FIG. 9 shows a state where a cap 5c is attached to one branch portion of the branch adapter 20 and the syringe 10 is attached to another branch portion. Additionally, as shown in FIG. 10, a liquid can be suctioned using two syringes, respectively, by connecting a syringe 10a different from the above-described syringe 10 to one branch portion of the branch adapter 20. Additionally, as shown in FIG. 11, the syringes 10 and 10a can also be similarly connected to the second connection port 5b via the branch adapter 20. In the present embodiment, if the branch adapter 20 is used after being attached to both the first connection port 5a and the second connection port 5b, a liquid can also be suctioned using four syringes. The number of branches of the branch adapter 20 may be three or more.

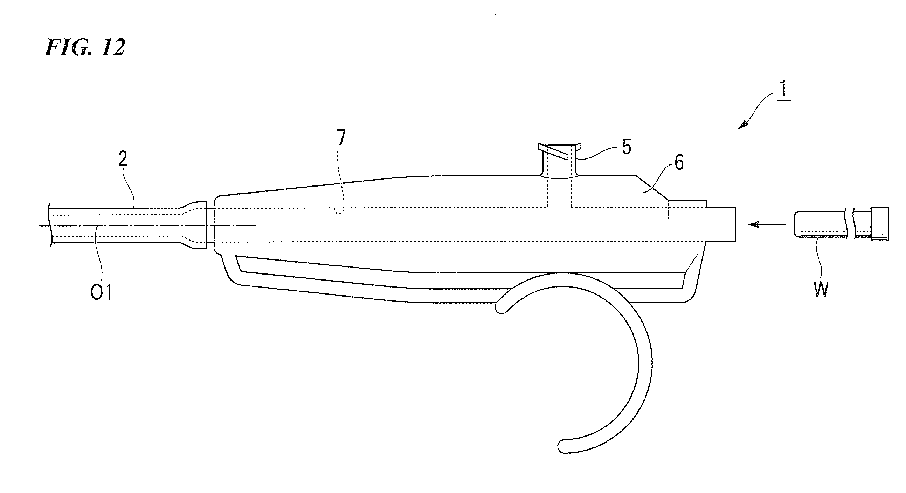

As shown in FIG. 12, the passage 7 formed in the operating portion 6 is a passage that has a central axis on an extension line obtained by extending the central axis O1 of the tube member 2 to the proximal end side, and has a diameter such that the stylet W can be inserted therethrough so as to be capable of advancing and retreating. Additionally, the passage 7 may have a taper shape whose diameter increases gradually as it goes to the proximal end side.

The connection port 5 provided in the operating portion 6 branches from the passage 7, and opens to an external surface of the operating portion 6.

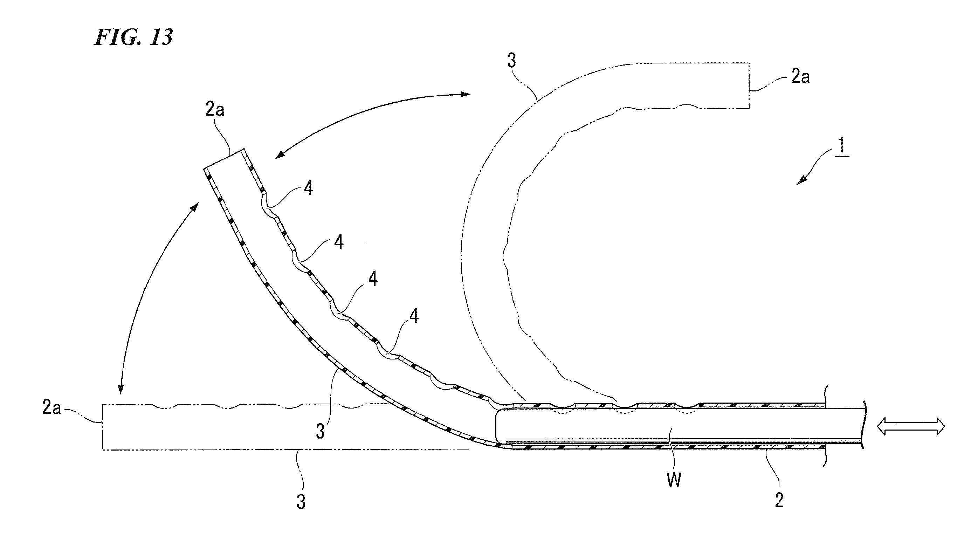

As shown in FIGS. 12 and 13, the stylet W, is configured so as to have higher rigidity than the tube member 2 such that the tube member 2 is brought into a linear state by the stylet W in a state where the stylet W is inserted through the bending portion 3 of the tube member 2. That is, a restoring force by which the stylet W is brought into a linear state is larger than a restoring force by which the bending portion 3 is brought into a bent state. When the stylet W and the tube member 2 are integrally inserted into the channel 102 (refer to FIG. 7) in a state where the stylet W is inserted through the tube member 2, the stylet W and the tube member 2 is bendable along the shape of the inner surface of the channel 102.

As shown in FIGS. 12 and 13, in the present modified example, the stylet W can be guided to the distal end 2a of the tube member 2 through the passage 7. At this time, the bending portion 3 is brought into a linear state by the stylet W inserted into the bending portion 3.

In the present modified example, the operator passes the stylet W to the distal end 2a of the tube member 2 via the passage 7 of the operating portion 6 before the tube member 2 is inserted through the channel 102. Accordingly, the bending portion 3 is bought into a linear state by the stylet W, and the rigidity thereof is made higher than that of the tube member 2 itself by the stylet W. For this reason, the distal end 2a and the bending portion 3 of the tube member 2 is easily inserted into the channel 102.

Additionally, in the bending portion 3, the distal end of the stylet W is advanced to the opening formed at the distal end 2a of the tube member 2 along the surface located on the outer side of the bend in the inner surface of the bending portion 3. In the case of the present modified example, the through holes 4 formed in the bending portion 3 open to the inner side of the bend. Thus, the distal end of the stylet W is not caught in the through holes 4, and the stylet W does not come out of the through holes 4.

After the distal end 2a of the tube member 2 is exposed from the distal end of the channel 102, the stylet W is removed from the tube member 2. Accordingly, the bending portion 3 is brought into the original bent state even by the restoring force of the bending portion 3.

Thereafter, a liquid in the body can be collected, similar to the above-described first embodiment.

### (Second Embodiment)

Next, an endoscopic device of a second embodiment of the present invention will be described.

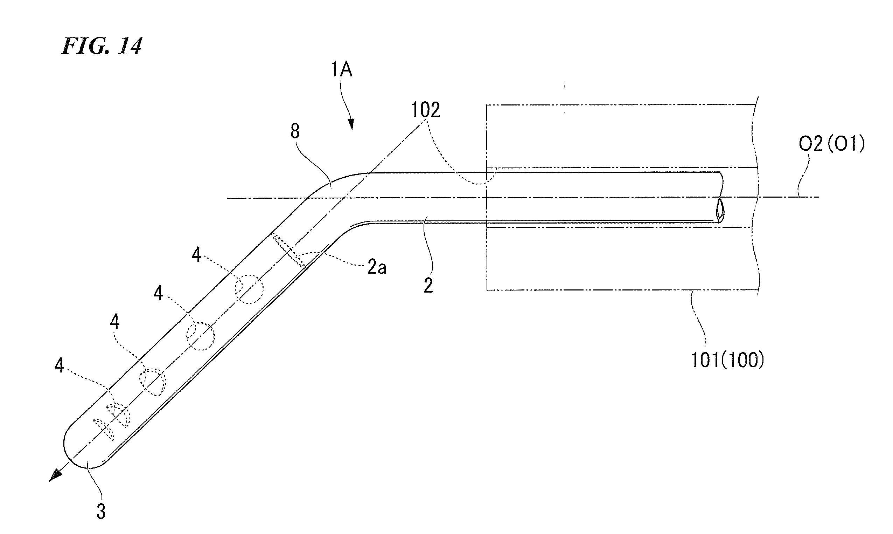

FIG. 14 is a side view showing the endoscopic device of the present embodiment. An endoscopic device 1A of the present embodiment is different from the first embodiment in that a curved portion 8 formed in a shape in which the central axis O1 of the tube member 2 is curved is provided further toward to the proximal end side than the bending portion 3 in the tube member 2.

The curved portion 8 is arranged at a position where the curved portion comes out of the distal end of the channel 102 in a state where the endoscopic device 1A is attached to the channel 102 of the endoscope 100.

By forming the curved portion 8, the bending portion 3 is directed to a direction that intersects the central axis 02 of the channel 102 in a state where the endoscopic device 1A is combined with the endoscope 100.

The operation of the endoscopic device 1A of the present embodiment will be described.

When the endoscopic device 1A is used, the operator inserts the tube member 2 into the channel 102 from its distal end, similar to the above-described first embodiment.

In a state where the distal end 2a of the tube member 2 protrudes from the distal end of the channel 102, the bending portion 3 and the curved portion 8 are restored to their original shapes, respectively.

Since the bending portion 3 is directed to a direction that intersects the central axis 02 of the channel 102 by the curved portion 8, even when there is not sufficient space where an insertion portion 101 of the endoscope 100 is moved, the depression X (refer to FIG. 2) can be formed more easily than the endoscopic device 1 of the above-described first embodiment.

In addition, in the case of the present embodiment, a large depression X can be formed by greatly setting the curvature of the curved portion 8 or greatly setting the length between the bending portion 3 and the curved portion 8.

### (Modified Example)

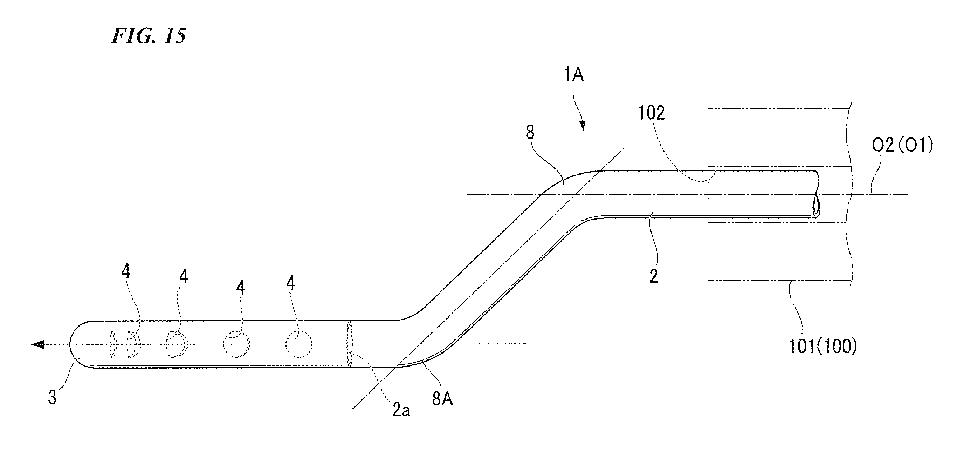

Next, a modified example of the endoscopic device 1A of the present embodiment will be described. FIG. 15 is a side view showing the configuration of the endoscopic device of the present modified example.

As shown in FIG. 15, in the present modified example, the endoscopic device 1A includes another curved portion 8A in addition to the curved portion 8. A proximal end side of the curved portion 8 and a distal end side of the curved portion 8A are made parallel to each other.

According to the configuration as in the present modified example, the orientation of the bending portion 3 of the present modified example can be brought into a orientation in which this bending orientation is moved parallel to the bending portion 3 described in the above-described first embodiment.

### (Third Embodiment)

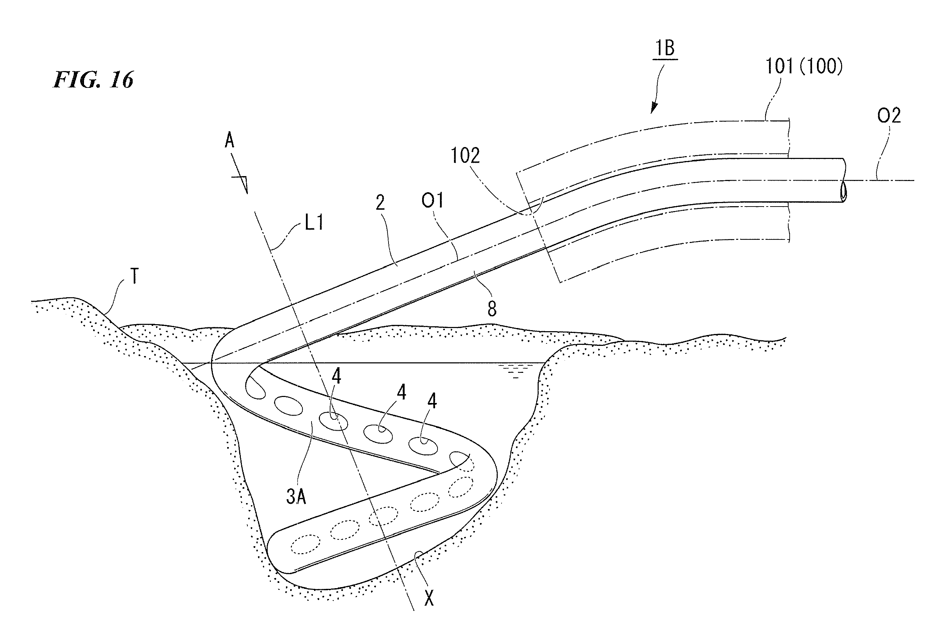

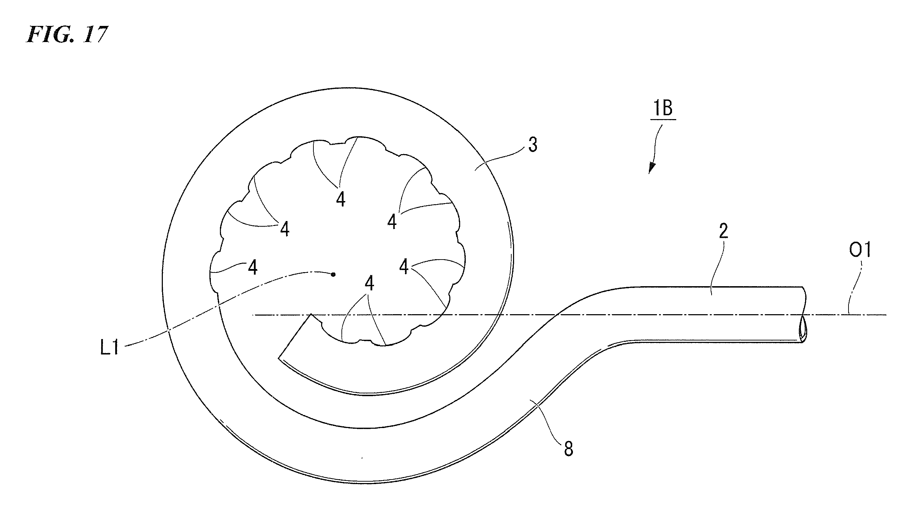

Next, an endoscopic device of a third embodiment of the present invention will be described. FIG. 16 is a side view showing the endoscopic device of the present embodiment. FIG. 17 is a view as seen from arrow A of FIG. 16.

As shown in FIGS. 16 and 17, the endoscopic device 1B of the present embodiment is different from the first and second embodiments in that this endoscopic device has a bending portion 3A formed in a conical coil shape.

The positional relationship between the central axis O1 of the tube member 2 closer to the proximal end side than the bending portion 3A formed in the conical coil shape and a centerline L1 of the bending portion 3A is an intersecting or twisted position. In other words, the centerline L1 of the bending portion 3A is arranged at a position where the centerline is twisted with respect to the central axis O1 of the tube member 2.

In the present embodiment, the openings of the through holes 4 formed in the bending portion 3A are directed to the inner side of the bend or to the centerline L1 side.

The operation of the endoscopic device 1B of the present embodiment will be described.

The bending portion 3A is brought into a state where the bending portion protrudes from the distal end of the channel 102 in a state where the endoscopic device 1B is inserted through the channel 102 of the endoscope 100.

At this time, the distal end of the bending portion 3A extends spirally toward a direction that intersects the central axis 02 of the channel 102. The operator presses the bending portion 3A against the surface of the tissue T and forms the depression X in the tissue T, similar to the above-described first embodiment (refer to FIG. 16).

The shape of the depression X formed in the tissue T by the bending portion 3A is a shape along an envelope that connects the external surface of the bending portion 3A. A liquid in the vicinity of the depression X is gathered in the depression X. Since the bending portion 3A is formed in a conical coil shape, the bending portion 3A may be slightly pushed back in the direction of the centerline L1 by the repulsive force of the tissue T.

Most of the through holes 4 formed in the bending portion 3A are located within the liquid without touching the tissue T inside the depression X. For this reason, the liquid can be suctioned through the through holes 4 and can be collected within the syringe11 (refer to FIG. 1).

As described above, according to the endoscopic device 1B of the present embodiment, a large depression X can be formed in the tissue T. Additionally, since the bending portion 3A deforms due to the repulsive force of the tissue T, the depression X can be formed without damaging the tissue T even in the tissue T where a hard portion and a soft portion coexist.

The positional relationship (positional relationship between the central axis O1 of the tube member 2 and the centerline L1 of the bending portion 3A) between the centerline L1 of the bending portion 3A to the central axis O1 of the tube member 2 may be intersecting or orthogonal.

### (Fourth Embodiment)

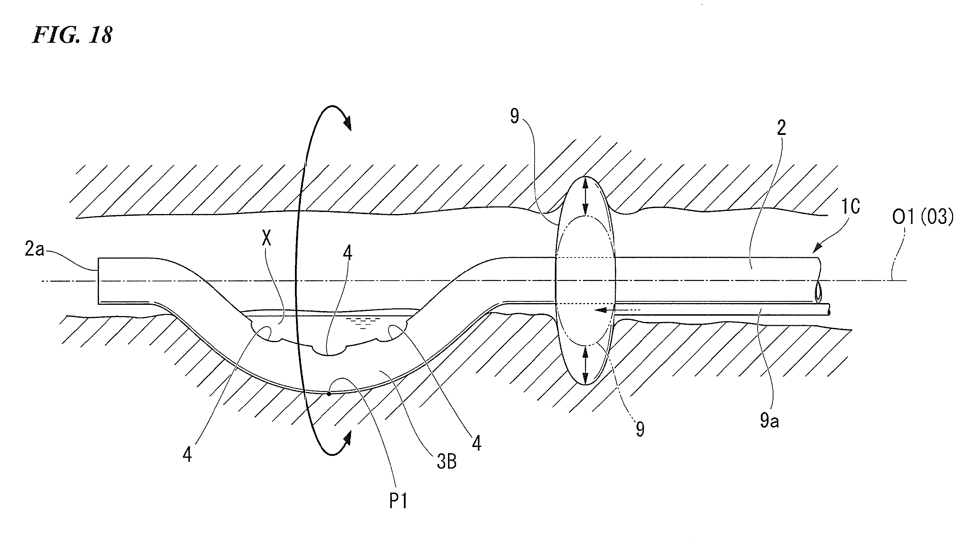

Next, an endoscopic device of a fourth embodiment of the present invention will be described. FIG. 18 is a side view showing the configuration and operation in use, of the endoscopic device of the present embodiment of the present invention.

As shown in FIG. 18, an endoscopic device 1C of the present embodiment has a bending portion 3B that has a shape different from the bending portion 3 and the bending portion 3A that are described in the above-described respective embodiments, and is different from the first to third embodiments in that a balloon 9 is provided on the proximal end side of the bending portion 3B.

A distal end portion and a proximal end portion of the bending portion 3B are located on the central axis O1 of the tube member 2, and the bending portion 3B is formed in a bent shape that has a peak P1 at a position that is eccentric by a predetermined distance from the central axis O1 of the tube member 2 in an intermediate portion. Additionally, in the present embodiment, the opening of the distal end 2a of the tube member 2 is closed. The above predetermined distance is set on the basis of the size of a tissue that is a target from which a liquid is collected. For example, when a liquid is collected within a lumen tissue T1, the predetermined distance is set to about a distance such that the peak P1 of the bending portion 3B presses the inner surface of the lumen tissue T1 in a state where the distal end portion and proximal end portion of the bending portion 3B are located on the central axis 03 of the lumen tissue T1, that is, to a dimension slightly larger than the radius of the lumen tissue T1.

The balloon 9 is formed from a stretchable member that inflates if a fluid flows thereinto. The balloon 9 is provided with an operation tube 9a that communicates with the inside of the balloon 9. The operation tube 9a extends to the proximal end 2b side parallel to the tube member 2.

The maximum diameter when a fluid flows into the balloon 9 is set on the basis of the size of a tissue that is a target from which a liquid is collected. For example, when a liquid is collected within the lumen tissue T1, the maximum diameter is set so as to be larger than the internal diameter of the lumen tissue T1 when the balloon 9 has inflated within the lumen tissue T1.

The operation of the endoscopic device 1C of the present embodiment will be described.

The endoscopic device 1C of the present embodiment is configured so that a liquid can be favorably collected within the lumen tissue T1. Specifically, when the endoscopic device 1C is used, the bending portion 3B is guided to a region where a liquid is collected within the lumen tissue T1. Subsequently, if the operator inflates the balloon 9, the balloon 9 inflates larger than the internal diameter of the lumen tissue T1, and the inner surface of the lumen tissue T1 is pressed. Accordingly, the lumen tissue T1 is brought into a blocked state by the balloon 9, and a portion closer to the proximal end side than the balloon 9 is brought into a state where a liquid in the body does not flow thereinto. Moreover, as the balloon 9 and the lumen tissue T1 abut against each other, the proximal end and distal end of the bending portion 3B are positioned on the central axis 03 of the lumen tissue T1, respectively.

The operator rotates the tube member 2 around the central axis O1 of the tube member 2 as a rotation center. If the proximal end of the tube member 2 is rotated around the central axis O1 outside the body, a rotative force is transmitted to the distal end of the tube member 2, and the bending portion 3B also rotate around the central axis O1 of the tube member 2. The surface directed to the outer side of the bend in the external surface of the bending portion 3B comes into contact with the inner surface of the lumen tissue T1, and the depression X is formed in this contact place. As the bending portion 3B rotates around the central axis O1, the position of the depression X on the inner surface of the lumen tissue T1 varies gradually, pleats are smoothed, and the liquid is collected.

If the liquid is collected in the depression X, the liquid can be collected within the syringe11 (refer to FIG. 1) through the through holes 4 that open to the inner side of the bend of the bending portion 3B, similar to the first embodiment.

According to the endoscopic device 1C of the present embodiment, a liquid can be efficiently collected while preventing the liquid from flowing into the proximal end side of the tube member 2.

Another balloon may be arranged at the distal end of the bending portion 3B in addition to the proximal end of the bending portion 3B. In this case, a region where the liquid is collected can be limited to between the proximal end side and the distal end side of the bending portion 3B. As a method of using a endoscopic device including the balloon 9 and the other balloon, for example, an example in which bile is collected from the inside of the duodenum to the outside of the body can be mentioned. That is, by arranging the base-end-side balloon 9 on the upstream side of duodenal papilla and arranging the other balloon on the downstream side of the duodenal papilla, bile can be prevented from flowing into other portions within the duodenum from the vicinity of the duodenal papilla.

While preferred embodiments of the present invention have been described and illustrated above, it should be understood that these are exemplary of the present invention and are not to be considered as limiting. Additions, omissions, substitutions, and other modifications can be made without departing from the concept of the present invention. The present invention is not to be considered as being limited by the foregoing description, and is limited only by the scope of the appended claims.

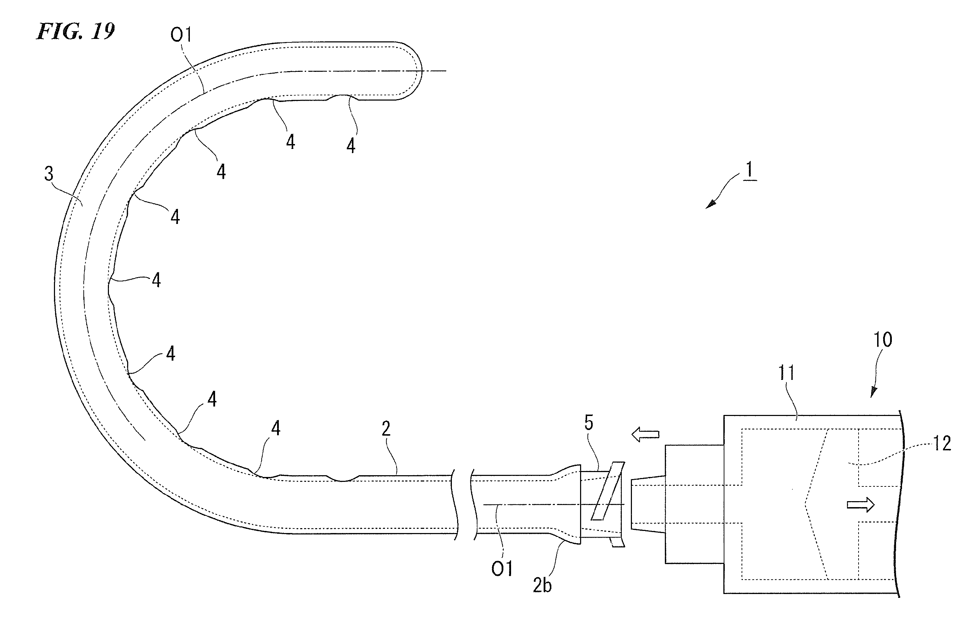

For example, the distal end 2a of the tube member 2 does not necessarily opens. For example, as shown in FIG. 19, a cap that closes the distal end 2a of the tube member 2 may be provided, or the tube member 2 may be crushed so as to close the distal end 2a of the tube member 2.

Additionally, the bending portion 3 may have a predetermined restoring force that bends the bending portion such that the through hole 4 is directed to the inner side of the bend of the bending portion 3.

Additionally, the bending portion 3 may be spirally formed.

### Industrial Applicability

According to the endoscopic device of the above respective aspects, the surface directed to the outer side of the bend in the external surfaces of the bending portion is pressed against the tissue, so that the depression can be formed in the tissue and a liquid can be collected through the through holes on the inner side of the bend. As a result, since a liquid can be collected in the depression, the liquid can be efficiently collected.

Moreover, since the through holes opens to the inner side of the bend of the bending portion, the openings of the through holes are not closed by the tissue, and a liquid can be efficiently collected. Additionally, according to the endoscopic device of the present embodiment, the tissue can also be prevented from being suctioned through the through holes.

### Reference Signs List

- O1:: CENTRALAXIS
- L1:: CENTERLINE
- W:: STYLET
- 1, 1A, 1B:: ENDOSCOPIC DEVICE
- 2:: TUBE MEMBER
- 3:: BENDING PORTION
- 4:: THROUGH HOLE
- 5:: CONNECTION PORT
- 7:: PASSAGE
- 8:: CURVED PORTION

## Claims

1. An endoscopic device comprising:
a longitudinal member which has a longitudinal axis;
a bending portion which is formed on a distal end side of the longitudinal member;
a lumen which is formed along the longitudinal axis of the longitudinal member;
a through hole which communicates with the lumen and opens toward an inner side of a bend of the bending portion.

2. The endoscopic device according to Claim 1,
wherein a connection port that is connected to a suction portion that suctions a liquid through an inside of the longitudinal member is attached to a proximal end side of the longitudinal member.

3. The endoscopic device according to Claim 2,
wherein the bending portion has a predetermined restoring force that bends the bending portion so that the through hole is directed to the inner side of the bend of the bending portion.

4. The endoscopic device according to Claim 2,
wherein the longitudinal member has openings at the distal end and the proximal ends, respectively,
wherein the proximal end of the longitudinal member is provided with an operating portion formed with a passage for inserting a stylet into the longitudinal member, and
wherein the connection port branches from the passage and opens to an external surface of the operating portion.

5. The endoscopic device according to Claim 3,
wherein the bending portion is spirally formed.

6. The endoscopic device according to Claim 3,
wherein the bending portion has the predetermined restoring force in order to press a tissue with restoring to a predetermined bend shape.

7. The endoscopic device according to Claim 3,
wherein the bending portion is provided on the distal end side of the longitudinal member, and
wherein the longitudinal member is provided with a curved portion formed such that a central axis of the longitudinal member is curved closer to the proximal end side than the bending portion.

8. The endoscopic device according to Claim 3,
wherein the longitudinal member has flexibility and is insertable through a channel of an endoscope.

9. The endoscopic device according to Claim 5,
wherein the bending portion is provided on the distal end side of the longitudinal member and is formed in a conical coil shape.

10. The endoscopic device according to Claim 9,
wherein a positional relationship between a central axis of the longitudinal member closer to the proximal end side than the bending portion and a centerline of the bending portion is an intersecting or twisted position.

11. The endoscopic device according to Claim 10,
wherein a balloon is provided on at least one of the proximal end side of the bending portion and the distal end side of the bending portion,
wherein the longitudinal member is inserted into a digestive tract, and
wherein the balloon inflates larger than the internal diameter of the lumen tissue by supplying a fluid thereinto.
